# EUROPEAN PATENT APPLICATION

(11) **EP 4 413 995 A1**
(43) Date of publication of application: **14.08.2024**
(21) Application number: 23305161.4
(22) Date of filing: 07.02.2023
(51) Int. Cl.: A61K 39/00, C07K 16/28

(54) **ANTI-CD123 CHIMERIC ANTIGEN RECEPTORS T CELLS FOR USE IN THE TREATEMENT OF AUTOIMMUNE DISEASES**

(71) Applicant: Université de Franche-Comté, 25030 Besançon (FR); Institut National de la Santé et de la Recherche Médicale, 75654 Paris Cedex 13 (FR); Etablissement français du sang, 93218 La Plaine Saint Denis Cedex (FR); Centre Hospitalier Régional Universitaire de Besançon, 25000 Besançon (FR)
(72) Inventor: GARNACHE OTTOU, Francine, 25030 Besançon (FR); ADOTEVI, Olivier, 25030 Besançon (FR); CAËL, Blandine, 25030 Besançon (FR); GALAINE, Jeanne, 25030 Besançon (FR); BOLE RICHARD, Elodie, 25030 Besançon (FR)
(74) Representative: Cabinet Netter

(57) **Abstract**

The invention is relative to An isolated chimeric antigen receptor (CAR) molecule comprising an antibody or antibody fragment which includes an anti-CD123 binding domain, a transmembrane domain, and an intracellular signaling domain comprising at least a stimulatory domain, and wherein said anti-CD123 binding domain comprises a heavy chain comprising a complementary determining region 1 (CDR1) having at least 90% identity to amino acid sequence SEQ ID NO : 1, a complementary determining region 2 (CDR2) having at least 90% identity to amino acid sequence SEQ ID NO : 2, and a complementary determining region 3 (CDR3) having at least 90% identity to amino acid sequence SEQ ID NO : 3, and a light chain comprising a complementary determining region 1 (CDR1) having at least 90% identity to amino acid sequence SEQ ID NO : 4, a complementary determining region 2 (CDR2) having at least 90% identity to amino acid sequence Serine-Threonine-Serine (STS), and a complementary determining region 3 (CDR3) having at least 90% identity to amino acid sequence SEQ ID NO : 5 for use in the treatment of an autoimmune disease.

## Description

The present invention is relative to anti-CD123 CAR-T cells for use in the treatment of autoimmune diseases. More particularly, the invention is relative to said CAR-T cells in diseases mediated by plasmacytoid dendritic cells (pDCs).

Autoimmune diseases are widely spread among human population all around the globe. Basically, autoimmune diseases are known to happen when the body's natural immune system can't tell the difference between healthy cells from the own body and unhealthy foreign germs and/or cells. As a consequence, the immune systems mistakenly attacks healthy body cells.

There are more than 80 types of autoimmune diseases. Some of them are known to be linked to plasmacytoid dendritic cells (pDCs) which are cells from the innate immune system that participate in the first line of defense against foreign germs and/or cells entering the body.

Plasmacytoid dendritic cells (pDCs) are known to be specialized in the production of type I interferon (IFN-I) in many autoimmune and inflammatory diseases such as lupus erythematosus, inflammatory myopathies (dermatomyositis, polymyositis), systemic scleroderma or psoriasis; cf. Ganguly, D. et al. Self-RNA-antimicrobial peptide complexes activate human dendritic cells through TLR7 and TLR8. J. Exp. Med. (2009); Bell, E. Plasmacytoid dendritic cells in psoriasis,
Nat. Rev. Immunol. (2007); and Albanesi, C., Scarponi, C., Bosisio, D., Sozzani, S. & Girolomoni, G. Immune functions and recruitment of plasmacytoid dendritic cells in psoriasis, Autoimmunity (2010).

More particularly, in pDCs linked diseases a strong infiltration by the pDCs and their secretion of IFN-I in the affected skin lesions and organs have been described in recent studies, cf. Huang X et al., Predominant role of plasmacytoid dendritic cells in stimulating systemic autoimmunity, Front. Immunol (2015); Tucci M et al, Glomerular accumulation of plasmacytoid dendritic cells in activate lupus nephritis : role of interleukin-18. Arthritis Rheum (2008); and Kafaja S et al., pDCs in lung and skin fibrosis in a bleomycin-indiced model and patients with systemic sclerosis, JCI Insight (2018).

The CD123 molecule is now known to be strongly expressed in pDCs. Moreover, the CD123 molecule seems to be almost specific to plasmacytoid dendritic cells; cf. Oon, S. et al., A cytotoxic anti-IL-3Rα antibody targets key cells and cytokines implicated in systemic lupus erythematosus, JCI Insight, (2016); and Bôle-Richard, E. et al., CD28/4-1BB CD123 CAR T cells in blastic plasmacytoid dendritic cell neoplasm, Leukemia (2020). This makes CD123 an interesting biomarker to target.

However, targeting biomarkers such as CD123 is generally difficult due to numerous technical hurdles such as specificity problems, transduction and proliferation efficiency, and cytotoxicity.

Recently, in another technical field (i.e. cancer research) a method that uses specifically engineered T cells (or T lymphocytes) has been developed; Singh, A. K. & McGuirk, J. P. CAR T cells: continuation in a revolution of immunotherapy. Lancet Oncol, (2020). These T cells comprise so called Chimeric Antigen Receptors commonly referred to as CAR (or CARs). CARs are able to fight cancer in that they are specifically directed against a target antigen expressed by tumor cells. Yet, the field of cancer research and the one of auto-immune disease are very different by nature and generally use radically different treatment methods.

The present invention improves the situation by using an anti-CD123 CAR-T cells as described in EP3753954 (application EP19305816, also published under WO2020254682) from the Applicant. The inventors have surprisingly discovered that CAR-T cells that have been engineered specifically to fight an aggressive cancer called Blastic Plasmacytoid Dendritic Cell Neoplasm (BPDCN) are able to improve immunotherapies in autoimmune diseases.

The present invention thus focuses on using a specific CAR-T Cell directed against CD123 in therapy against autoimmune diseases.

To that end, an object of the present invention is an isolated chimeric antigen receptor (CAR) molecule comprising an antibody or antibody fragment which includes an anti-CD123 binding domain, a transmembrane domain, and an intracellular signaling domain comprising at least a stimulatory domain, and wherein said anti-CD123 binding domain comprises a heavy chain comprising a complementary determining region 1 (CDR1) having at least 90% identity to amino acid sequence SEQ ID NO : 1 (GYSITSDYT), a complementary determining region 2 (CDR2) having at least 90% identity to amino acid sequence SEQ ID NO : 2 (ISFSGST), and a complementary determining region 3 (CDR3) having at least 90% identity to amino acid sequence SEQ ID NO : 3 (ARGLDY), and a light chain comprising a complementary determining region 1 (CDR1) having at least 90% identity to amino acid sequence SEQ ID NO : 4 (SSISSSY), a complementary determining region 2 (CDR2) having at least 90% identity to amino acid sequence Serine-Threonine-Serine (STS), and a complementary determining region 3 (CDR3) having at least 90% identity to amino acid sequence SEQ ID NO : 5 (HQLHRSPWT) for use in the treatment of an autoimmune disease.

According to an embodiment of the invention, the autoimmune disease is selected from the group consisting of Cutaneous Lupus Erythemathosus, Dermatomyositis, Psoriasis and systemic sclerosis.

According to another embodiment of the invention, the anti-CD123 binding domain is selected from the group consisting of an antibody, a Fv, a scFv, a Fab, or another antibody fragment, preferably a scFv.

According to another embodiment of the invention, the intracellular signaling domain is CD3-zeta (CD3ζ), optionally comprising a costimulatory domain selected from the group consisting of CD28, 4.1BB, Inducible T-cell COStimulator (ICOS), OX-40 or a combination thereof.

According to an embodiment, the isolated chimeric antigen receptor of the invention has having at least 90% identity, preferentially 100% identity, to nucleic acid sequence SEQ ID NO : 6.

According to another aspect, the object of the invention is an expression vector comprising a nucleic acid molecule having SEQ ID NO : 6, wherein the vector is selected from the group consisting of a DNA, a RNA, a plasmid, a lentivirus vector, an adenoviral vector, or a retrovirus vector for use in the treatment of an autoimmune disease. According to an embodiment, the autoimmune disease is selected from the group consisting of Cutaneous Lupus Erythemathosus, Dermatomyositis, Psoriasis and systemic sclerosis.

According to another aspect, the object of the invention is an engineered immune cell comprising the nucleic acid molecule having SEQ ID NO : 6 or the vector defined above for use in the treatment of an autoimmune disease. According to an embodiment, autoimmune disease is selected from the group consisting of Cutaneous Lupus Erythemathosus, Dermatomyositis, Psoriasis and systemic sclerosis.

According to another aspect, the object of the invention is a pharmaceutical composition comprising an isolated chimeric antigen receptor (CAR) as defined above, an expression vector as defined above, and/or an engineered immune cell as defined above and a pharmaceutical excipient for use in the treatment of an autoimmune disease. According to an embodiment, autoimmune disease is selected from the group consisting of Cutaneous Lupus Erythemathosus, Dermatomyositis, Psoriasis and systemic sclerosis.

Other features and advantages of the invention will stand out and/or become clear upon reading the following description, which comprises specific examples given in an illustrative and non-limiting manner, as well as from the drawings in which:
Figure 1 shows the nucleotide sequence and the amino acid sequence of the complementary determining region 1 (CDR1), complementary determining region 2 (CDR2) and complementary determining region 3 (CDR3) of both the heavy chain and the light chain of the antibody of the invention;
Figure 2 shows the nucleotide sequence and the amino acid sequence of framework region 1 (FR1), framework region 2 (FR2), framework region 3 (FR3) and framework region 4 (FR4) of the heavy chain of the antibody of the invention;
Figure 3 shows the nucleotide sequence and the amino acid sequence of framework region 1 (FR1), framework region 2 (FR2), framework region 3 (FR3) and framework region 4 (FR4) of the light chain of the antibody of the invention;
Figure 4 shows the consensus amino acid sequence of the light chain and the consensus amino acid sequence of the heavy chain of a the antibody of the invention;
Figure 5 shows a nucleic acid sequence of a chimeric antigen receptor of the invention; and
Figure 6 shows results regarding the anti-CD123 CAR-T of the invention on different blood samples.

The drawings and the description herein contain, for the most part, elements of definite nature. Therefore, description and drawings not only are being used to better understand the present invention, but also to contribute to the definition therefor, when appropriate.

In the present description, the term "and/or" should be interpreted as encompassing that one or more of the cases it connects may occur. For example, the wording "proteins or protein sequences can be prepared using standard recombinant and/or synthetic methods" indicates that proteins or protein sequences can be prepared using both standard recombinant and synthetic methods, or that proteins or protein sequences can be prepared using standard recombinant methods or indicates that proteins, or that proteins or protein sequences can be prepared using synthetic methods. More generally, the term "and/or" as used in a phrase such as "A and/or B" is intended to include "A and B", "A or B", "A" and "B". Likewise, the term "and/or" as used in a phrase such as "A, B, and/or C" is intended to encompass each of the following embodiments: A, B, and C; A, B, or C; A or C; A or B; B or C; A and C; A and B; B and C; A (alone); B (alone); and C (alone).

The term "comprising" is to be interpreted as encompassing all specifically mentioned features as well optional, additional, unspecified ones. More generally, the terms "comprise", "comprises" and "comprising" will be understood to imply the inclusion of a stated step or element or group of steps or elements but not the exclusion of any other step or element or group of steps or elements. The term "consisting of" is to be interpreted as no features other than the specifically mentioned features are present. Further, the indefinite article "a" or "an" does not exclude a plurality.

When reference is made throughout the present description to "one embodiment", "an embodiment", "a particular embodiment", "a certain embodiment", "an additional embodiment", "another embodiment" or "a further embodiment" or "one aspect", "an aspect", "a particular aspect", "a certain aspect", "an additional aspect", "another aspect" or "a further aspect" combinations thereof means that a particular feature, structure or characteristic described in connection with the embodiment is included in at least one embodiment of the present invention. The articles "a", "an" and "the" are used herein to refer to one or to more than one (i.e. to at least one) of the grammatical object of the article.

The term "gene" means a DNA sequence (nucleic acid sequence or nucleotide sequences) that codes for a particular sequence of amino acids. A gene comprises all or part of one or more proteins or enzymes, and may include regulatory DNA sequences (such as promoter sequences) which determine at least partially the conditions under which the gene is expressed. Some genes, which are not structural genes, may be transcribed from DNA to RNA, but are not translated into an amino acid sequence. Other genes may function as regulators of structural genes or as regulators of DNA transcription. In particular, the term gene may be intended for the genomic sequence encoding a protein, i.e. a sequence comprising regulator, promoter, intron and exon sequences.

The terms "identical" or percent (%) "identity" in the context of two or more nucleic acids sequences or amino acid sequences, refer to two or more sequences or subsequences that are the same or have a specified percentage of nucleotides or amino acid residues that are the same, when compared and aligned (introducing gaps, if necessary) for maximum correspondence, not considering any conservative amino acid substitutions as part of the sequence identity. The percent identity can be measured using sequence comparison software or algorithms or by visual inspection. Various algorithms and software are known in the art that can be used to obtain alignments of amino acid or nucleotide sequences. One such non-limiting example of a sequence alignment algorithm is the algorithm described in Karlin et al., Proc. Natl. Acad. Sci. 87:2264-2268, 1990, as modified in Karlin et al., Proc. Natl. Acad. Sci. 90:5873-5877, 1993, and incorporated into the NBLAST and XBLAST programs (Altschul et al., Nucleic Acids Res. 25:3389-3402, 1991). Gapped BLAST can also be used as described in Altschul et al., Nucleic Acids Res. 25:3389-3402, 1997; BLAST-2, WU- BLAST-2 (Altschul et al., Methods in Enzymology 266:460-480, 1996), ALIGN, ALIGN-2 (Genentech, South San Francisco, California) or Megalign (DNASTAR) are additional publicly available software programs that can be used to align sequences. Further, the algorithm of Needleman and Wunsch (J. Mol. Biol. (48):444-453, 1970) can be used to determine the percent identity between two amino acid sequences (e.g., using either a Blossum 62 matrix or a PAM250 matrix, and a gap weight of 16, 14, 12, 10, 8, 6, or 4 and a length weight of 1, 2, 3, 4, 5). Alternatively, the percent identity between nucleotide or amino acid sequences can be determined using the algorithm of Myers and Miller (CABIOS, 4: 11-17, 1989). For example, the percent identity can be determined using the ALIGN program (version 2.0) and using a PAM120 with residue table, a gap length penalty of 12 and a gap penalty of 4. Appropriate parameters for maximal alignment by particular alignment software can be determined by one skilled in the art. Usually, the default parameters of the alignment software are used.

A sequence "at least 85% identical to a reference sequence", or "something having at least 85% identity to a reference sequence" is a sequence having, on its entire length, 85%, or more, in particular 90%, 91 %, 92%, 93%, 94%, 95%, 96%, 97%, 98%, 99% or 100% sequence identity with the entire length of the reference sequence. In the context, the present description, the "percentage of identity" is calculated using a pairwise alignment (i.e. the two sequences are compared over their entire length). As mentioned above, methods for comparing the identity of two or more sequences are well known in the art. For instance, bioinformatics tools or programs, such as EMBOSS Needle available on the ebi.ac.uk Website, may be used to generate pairwise sequence alignments of the present invention. Commonly, many bioinformatics tools and programs use the Needleman-Wunsch algorithm (Needleman and Wunsch, 1970 J. Mol. Biol. 48:443-453) to find the optimum alignment (including gaps) of two sequences when considering their entire length. However, other algorithms may be implemented in a variety of bioinformatics tools or programs.

More generally, for the purposes of the present invention, the "sequence identity" or "sequence homology" is calculated by comparing two aligned sequences in a comparison window. The sequence alignment enables to determine the number of positions (nucleotides or amino acids) common to both of the two sequences in the comparison window. The number of common positions is then divided by the total number of positions in the comparison window and multiplied by hundred to obtain the percentage of homology. The determination of the percentage of sequence identity can be done manually or by using well-known bioinformatics computer programs. The percentage of identity between two polypeptides, in accordance with the invention, can be calculated using the EMBOSS: needle (global) program with a "Gap Open" parameter equal to 10.0, a "Gap Extend" parameter equal to 0.5, and a Blosum62 matrix.

Proteins, or a part thereof, having (or consisting of) an amino acid sequence "at least 80%, 85%, 90%, 95%, 96%, 97%, 98% or 99% identical" to a reference sequence may comprise mutations such as deletions, insertions and/or substitutions compared to the reference sequence.

In case of substitutions, the protein consisting of an amino acid sequence at least 80%, 85%, 90%, 95%, 96%, 97%, 98% or 99% identical to a reference sequence may correspond to a homologous sequence derived from another species than the reference sequence (i.e. "homology"). Substitutions of amino acids may be conservative or non-conservative. Conservative substitutions are substitutions wherein one amino acid is substituted for another amino acid with a similar side chain, i.e. similar structural and/or similar chemical properties. In this regard, conservative substitutions may occur between:
- amino acids with non-polar side chains: glycine (Gly), alanine (Ala), valine (Val), leucine (Leu), isoleucine (Ile), proline (Pro), phenylalanine (Phe), methionine (Met), tryptophan (Trp);
- amino acids with uncharged polar side chains: asparagine (Asn), glutamine (Gln), serine (Ser), threonine (Thr), tyrosine (Tyr), cysteine (Cys);
- amino acids with acidic side chains: aspartic acid (Asp), glutamic acid (Glu);
- amino acids with basic side chains: lysine (Lys), arginine (Arg), histidine (His);
- amino acids with beta-branched side chains: threonine (Thr), valine (Val), isoleucine (Ile);
- amino acids with aromatic side chains: tyrosine (Tyr), phenylalanine (Phe), tryptophan (Trp) histidine (His). Non-conservative substitutions may occur randomly between the above. Other definitions may be given to conservative or non-conservative substitutions depending on the positions of individual amino acids within the tridimensional shape of the protein.

The techniques of the invention uses, unless indicated otherwise, are methods of chemistry, biochemistry, organic chemistry, molecular biology, microbiology, recombinant DNA techniques, genetics, genetic and/or biotechnological cell engineering, immunology, and cell biology that are known by the skilled person. When needed, such methods are described for the purpose of illustration. Most of those methods and other techniques are described in literature. See, e.g., Sambrook, et at., Molecular Cloning: A Laboratory Manual (3rd Edition, 2001); Sambrook, et at., Molecular Cloning: A Laboratory Manual (2nd Edition, 1989); Maniatis et al., Molecular Cloning: A Laboratory Manual (1982); Ausubel et al., Current Protocols in Molecular Biology (John Wiley and Sons, updated July 2008); Short Protocols in Molecular Biology: A Compendium of Methods from Current Protocols in Molecular Biology, John Wiley and Sons, 4th Ed. 1999; DNA Cloning: A Practical Approach, vol. I & II (IRL Press, Oxford, 1985); Techniques for the Analysis of Complex Genomes, Anand, Academic Press, 1992; Transcription and Translation, B. Names & S. Higgins, 1984; A Practical Guide to Molecular Cloning, Perbal, 1984; Antibodies, Harlow and Lane, Cold Spring Harbor Laboratory Press, Cold Spring Harbor, 1998; Current Protocols in Immunology, Q. E. Coligan, A. M. Kruisbeek, D. H. Margulies, E. M. Shevach and W. Strober, 1991; Annual Review of Immunology. Unless defined otherwise, all technical and scientific terms used herein have the same meaning as commonly understood by those of ordinary skill in the art in the field of the invention. Although any methods and materials similar or equivalent to those described herein can be used in the practice or testing of the present invention, preferred embodiments of compositions, methods and materials are described herein.

As understood by the skilled person and as described herein, an "antibody", also called "immunoglobulin" comprises two heavy chains and two light chains. Each heavy chain consists of a variable region and a first, second, and third constant regions, while each light chain consists of a variable region and a constant region, two heavy chains are linked to each other by disulfide bonds wherein each heavy chain is linked to a light chain by a disulfide bond.

In mammals, there are two types of light chain, lambda (I) and kappa (k). Further, there are five main heavy chain classes (or isotypes) classified as a, d, e, y, and m which determine the functional activity of an antibody molecule: IgA, IgD, IgE, IgG, and IgM.

Each chain contains distinct sequence domains. The light chain includes two domains or regions, a variable domain (VL) and a constant domain (CL). The heavy chain includes four domains, a variable domain (VH) and three constant domains (CH1, CH2 and CH3, collectively referred to as CH). More precisely the complete antibody forms a "Y" shape. The base or stem of the Y consists of the second and third constant regions (and for IgE and IgM, the fourth constant region) of two heavy chains bound together by disulfide bonds, sometimes called inter-chain disulfide bonds forming the hinge. Heavy chains y, and d have a constant region composed of three tandem (inline) Ig domains, and a hinge region for added flexibility; heavy chains m and e have a constant region composed of four immunoglobulin domains. The second and third constant regions are referred to as "CH2 domain" and "CH3 domain", respectively. Each arm or branch of the Y includes the variable region and first constant region of a single heavy chain bound to the variable and constant regions of a single light chain. The variable regions of the light and heavy chains are responsible for antigen binding. More generally, the variable regions of both light (VL) and heavy (VH) chains determine binding recognition and specificity to the antigen. The constant region domains of the light (CL) and heavy (CH) chains confer important biological properties such as antibody chain association, secretion, trans-placental mobility, complement binding, and binding to Fc receptors (FcR). The Fv fragment is the N-terminal part of the Fab fragment of an immunoglobulin and consists of the variable portions of one light chain and one heavy chain. The specificity of the antibody resides in the structural complementarity between the antibody combining site and the antigenic determinant. Antibody combining sites are made up of residues that are primarily from the hypervariable or complementarity determining regions (CDRs). Occasionally, residues from non-hypervariable or framework regions (FR) influence the overall domain structure and hence the combining site. Complementarity Determining Regions or CDRs refer to amino acid sequences that together define the binding affinity and specificity of the natural Fv region of a native immunoglobulin binding site. The light and heavy chains of an immunoglobulin each have three CDRs, commonly designated CDR1, CDR2 and CDR3, or more precisely designated CDR1-L, CDR2-L, CDR3-L and CDR1-H, CDR2-H, CDR3-H, respectively. Other nomenclatures can be found in literature Thus, the CDRs located in the variable domain of the heavy chain of the antibody can be referred to as CDRH 1, CDRH2, and CDRH3, whereas the CDRs located in the variable domain of the light chain of the antibody can be referred to as CDRL1, CDRL2, and CDRL3. Antibodies with different specificities (i.e., different combining sites for different antigens) have different CDRs. A conventional antibody antigen-binding site, therefore, includes six CDRs, comprising the CDR set from each of a heavy and a light chain V region.

As mentioned above, light and heavy chain variable regions contain so called "framework" region that interrupted by the hypervariable CDR regions. The CDRs can be defined or identified by methods, such as by sequence according to Kabat et *al.,* Wu, TT and Kabat, E. A., J Exp Med, 132(2):211-50, 1970; Borden, P. and Kabat E. A., PNAS, 84: 2440-2443, 1987; Kabat et al., Sequences of Proteins of Immunological Interest, U.S. Department of Health and Human Services, 1991 - or by structure according to Chothia et *al. -* Choithia, C. and Lesk, A.M., J Mol. Biol., 196(4) : 901-917, 1987; Choithia, C. et al., Nature, 342: 877 - 883, 1989.

The sequences of the framework regions of different light or heavy chains are relatively conserved within a species, such as humans. More generally, "Framework Regions" (FRs) are relatively conserved among different immunoglobulins in a single species. The framework region of an antibody, which is somewhat the combination of all framework regions of the constituent light and heavy chains, serves to position and align the CDRs in three-dimensional space, i.e. when the antibody is folded and active. The CDRs are primarily responsible for binding to an epitope of an antigen. As mentioned above, the CDRs of each chain are referred to as CDR1, CDR2, and CDR3. In fact, the CDRs are numbered sequentially starting from the N-terminus. The light and heavy chains of an immunoglobulin each have four FRs, designated FR1, FR2, FR3 and FR4, or more precisely for the light chain FR1-L, FR2-L, FR3-L, FR4-L, and for the light chain FR1-H, FR2-H, FR3-H, FR4-H, respectively. Accordingly, the light chain variable domain may thus be designated as (FR1-L)-(CDR1-L)-(FR2-L)-(CDR2-L)-(FR3-L)-(CDR3-L)-(FR4-L) and the heavy chain variable domain may thus be designated as (FR1-H)-(CDR1-H)-(FR2-H)-(CDR2-H)-(FR3-H)-(CDR3-H)-(FR4-H). Knowing the amino acid sequence of the CDRs one skilled in the art can easily determine the framework regions FR1 -L, FR2-L, FR3-L, FR4-L and/or FR1-H, FR2-H, FR3-H, FR4-H.

References in the present description to "V_{H}" or "VH" refer to the variable region of an immunoglobulin heavy chain, including that of an antibody, Fv, scFv, Fab, or other antibody fragment as disclosed herein. References to "V_{L}" or "VL" refer to the variable region of an immunoglobulin light chain, including that of an antibody, Fv, scFv, dsFv, Fab, or other antibody fragment as disclosed herein.

As used herein, the term "antibody" or "immunoglobulin" mainly denotes conventional antibodies, particularly monoclonal antibodies, and fragments thereof, as well as single domain antibodies and fragments thereof, in particular variable heavy chain of single domain antibodies, and further denotes chimeric antibodies, humanized, bispecific or multispecific antibodies. The antibody is preferably a human antibody, a murine antibody, or a humanized antibody.

A "monoclonal antibody" or "mAb" is an antibody produced by a single clone of B lymphocytes or by a cell into which the heavy and the light chain genes of a single antibody have been transfected. Monoclonal antibodies are produced by methods well known in the art, for instance by making hybrid antibody-forming cells from a fusion of myeloma cells with immune spleen cells or by recombination techniques, i.e. production by protein engineering. Monoclonal antibodies include humanized monoclonal antibodies. More generally, a monoclonal antibody is a molecule of a single amino acid composition that is directed against a specific antigen, and is not to be construed as requiring production of the antibody by any particular method other than those known in the art.

The term "chimeric antibody" refers to an engineered antibody which in its broadest sense contains one or more regions from one antibody and one or more regions from one or more other antibodies. In particular a chimeric antibody comprises a VH domain and a VL domain of an antibody derived from a non-human animal, in association with a CH domain and a CL domain of another antibody, in particular a human antibody. As the non-human animal, any animal such as mouse, rat, hamster, rabbit or the like can be used. A chimeric antibody may also denote a multispecific antibody having specificity for at least two different antigens.

The term "antibody" or "immunoglobulin" also includes the meaning of "single domain antibodies" which have been more recently described and developed. Single domain antibodies are antibodies whose complementary determining regions (CDRs) are part of a single domain polypeptide. Examples of single domain antibodies include heavy chain antibodies, antibodies naturally devoid of light chains, single domain antibodies derived from conventional four-chain antibodies, as well as engineered single domain antibodies. Single domain antibodies may be derived from any species, especially from mouse, human or rabbit. Single domain antibodies may be naturally occurring single domain antibodies known as heavy chain antibody devoid of light chains. In particular, *Camelidae* species (e.g. camel, dromedary, llama, alpaca and guanaco) produce heavy chain antibodies naturally devoid of light chain.

The variable heavy chain of these single domain antibodies devoid of light chains are known in the art as "VHH" or "nanobody". Similar to conventional VH domains, VHHs contain four framework regions (FRs) and three complementary determining regions (CDRs). One particular advantage of Nanobodies over conventional antibodies is that they are about ten times smaller than IgG molecules. Consequently, properly folded functional nanobodies can be produced by in vitro expression with a high yield. Furthermore, the art describes nanobodies as being very stable, and resistant to the action of proteases. The properties and production of nanobodies have been reviewed for instance in Harmsen and De Haard HJ, Appl. Microbiol. Biotechnol., 2007, Nov, 77(1):13-22.

The term antibodies also include antigen binding fragments thereof. Such "fragments" comprise a portion of an intact antibody, in particular the antigen binding region or variable region of the intact antibody. Examples of antibody fragments include Fab' fragments, F(ab)'2 fragments, Fv, single chain Fv proteins ("scFv") and portions of full length antibodies responsible for antigen binding, diabodies, bispecific and multispecific antibodies formed from antibody fragments. A fragment of a conventional antibody may also be a single domain antibody, such as a heavy chain antibody or VHH. The term "Fab" denotes an antibody fragment having a molecular weight of about 50,000 and antigen binding activity, in which about a half of the N-terminal side of H chain and the entire L chain, among fragments obtained by treating IgG with a protease, papaine, are bound together through a disulfide bond. The term "F(ab')2" refers to an antibody fragment having a molecular weight of about 100,000 and antigen binding activity, which is slightly larger than the Fab bound via a disulfide bond of the hinge region, among fragments obtained by treating IgG with a protease, pepsin. "Single-chain Fv" or "scFv" antibody fragments comprise the VH and VL domains of antibody, wherein these domains are present in a single polypeptide chain and in either orientation (e.g. VL-VH or VH-VL). Single chain may be cloned form the V region genes of a hybridoma specific for a desired target. The production of such hybridomas has become routine. A technique which can be used for cloning the variable region heavy chain (VH) and variable region light chain (VL) has been described, for example, in Orlandi et al., PNAS, 1989, 86: 3833-3837. Usually, the scFv polypeptide further comprises a polypeptide linker between the VH and VL domains which enables the scFv to form the desired structure for antigen binding. More specifically, a single chain Fv ("scFv") polypeptide is a covalently linked VH::VL heterodimer which is usually expressed from a gene fusion including VH and VL encoding genes linked by a peptide-encoding linker. In some embodiments, human scFv fragment of include CDRs that are held in appropriate conformation, in particular by using gene recombination techniques. Divalent and multivalent antibody fragments can form either spontaneously by association of monovalent scFvs, or can be generated by coupling monovalent scFvs by a peptide linker, such as divalent sc(Fv)2. "dsFv" is a VH::VL heterodimer stabilized by a disulphide bond. "(dsFv)2" denotes two dsFv coupled by a peptide linker. The term "bispecific antibody" or "BsAb" typically denotes an antibody, which combines the antigen-binding sites of two antibodies within a single molecule. Thus, BsAbs are able to bind two different antigens simultaneously. Genetic engineering has been used with increasing frequency to design, modify, and produce antibodies or antibody derivatives with a desired set of binding properties and effector functions as described for instance in EP2050764A1. The term "multispecific antibody" denotes an antibody that combines the antigen-binding sites of two or more antibodies within a single molecule. The term "diabodies" refers to small antibody fragments with two antigen-binding sites, which fragments comprise a heavy-chain variable domain (VH) connected to a light-chain variable domain (VL) in the same polypeptide chain (VH-VL). By using a linker that is too short to allow pairing between the two domains on the same chain, the domains are forced to pair with the complementary domains of another chain and create two antigen-binding sites.

The present invention provides immune effector cells genetically engineered with vectors designed to express Chimeric Antigen Receptors (CARs) that redirect cytotoxicity to cells and/or molecules implicate in auto-immune diseases. CARs are molecules that combine antibody-based specificity for a target antigen (e.g. autoimmune disease related antigen) with a T cell receptor-activating intracellular domain to generate a chimeric protein that exhibits a specific cellular immune activity. As used herein, the term, "chimeric" describes being composed of parts of different proteins or DNAs from different origins. The main characteristic of CARs are their ability to redirect immune effector cell specificity, thereby triggering proliferation, cytokine production, phagocytosis or production of molecules that can mediate cell death of the target antigen expressing cell in a major histocompatibility (MHC) independent manner, exploiting the cell specific targeting abilities of monoclonal antibodies, soluble ligands or cell specific co-receptors.

As used herein, the terms "binding domain", "extracellular binding domain", "antigen-specific binding domain", and "extracellular antigen specific binding domain", are used interchangeably and provide a CAR with the ability to specifically bind to the target antigen of interest. A binding domain may comprise any protein, polypeptide, oligopeptide, or peptide that possesses the ability to specifically recognize and bind to a biological molecule, such as a cell surface receptor or a protein implicated in auto-immune diseases, lipid, polysaccharide, or other cell surface target molecule, or component thereof. A binding domain includes any naturally occurring, synthetic, semi-synthetic, or recombinantly produced binding partner for a biological molecule of interest. The terms "specific binding affinity" or "specifically binds" or "specifically bound" or "specific binding" or "specifically targets" as used herein, describe binding of one molecule to another at greater binding affinity than background binding. A binding domain (or a CAR comprising a binding domain or a fusion protein containing a binding domain) "specifically binds" to a target molecule if it binds to or associates with a target molecule with an affinity or Ka (i.e. an equilibrium association constant of a particular binding interaction with units of 1/M) of, for example, greater than or equal to about 10⁵ M⁻¹. Affinities of binding domain polypeptides and CAR proteins according to the present disclosure can be readily determined using conventional techniques like competitive ELISA (enzyme-linked immunosorbent assay). Further the binding quality related to the affinity may be determined, quantified and qualified the so called K_{D} Value. The K_{D} and correlates to antibody affinity and sensitivity. K_{D} is the equilibrium dissociation constant, a ratio of k_{off}/kₒₙ, between the antibody and its antigen. K_{D} and affinity are inversely related. The K_{D} value relates to the concentration of antibody (the amount of antibody needed for a particular experiment) and so the lower the K_{D} value (lower concentration) and thus the higher the affinity of the antibody.K_{D} is the ratio of the antibody dissociation rate (k_{off}), how quickly it dissociates from its antigen, to the antibody association rate (kₒₙ) of the antibody, how quickly it binds to its antigen. The K_{D} values of the present description were determined by measuring the kₒₙ and k_{off} rates of a specific antibody/antigen interaction and then using a ratio of these values to calculate the K_{D} value. More generally, the affinity of an antibody is the strength of binding of a single molecule to its ligand. It is typically measured and reported by the equilibrium dissociation constant (K_{D}), which is used to evaluate and rank order strengths of bimolecular interactions. The binding of an antibody to its antigen is a reversible process, and the rate of the binding reaction is proportional to the concentrations of the reactants. At equilibrium, the rate of [antibody]|[antigen] complex formation is equal to the rate of dissociation into its components [antibody]+[antigen]. The measurement of the reaction rate constants can be used to define an equilibrium or affinity constant (1/ K_{D}). In short, the smaller the K_{D} value the greater the affinity of the antibody for its target.

The binding domain of the CAR is commonly followed by one or more "hinge regions". Hinge regions play a role in positioning the antigen binding domain away from the effector cell surface to enable proper cell-to-cell contact, antigen binding and activation. A CAR can have one or more hinge regions between the binding domain and the transmembrane domain. As described in the art hinge regions may be derived from a natural, synthetic, semi-synthetic, or recombinant source.

The "transmembrane domain" is a CAR portion that fuses the extracellular binding portion and intracellular signaling domain. The transmembrane domain anchors the CAR to the plasma membrane of the immune effector cell. As described in the art transmembrane regions may be derived from a natural, synthetic, semi-synthetic, or recombinant source.

In preferred embodiments, CARs of the invention comprise an intracellular signaling domain. An "intracellular signaling domain" refers to the part of a CAR that participates in transducing the message of effective CAR binding to the target antigen into the interior of the immune effector cell to elicit effector cell function, e.g. activation, cytokine production, proliferation and cytotoxic activity, including the release of cytotoxic factors to the CAR-bound target cell, or other cellular responses elicited with antigen binding to the extracellular CAR domain. The "effector function" refers to a specialized function of the cell. Effector function of a T cell may be cytolytic activity or activity including the secretion of cytokine. Consequently, in the present description an "intracellular signaling domain" refers to the portion of a protein which transduces the signal of the "effector function" which induces the cell to perform a specialized function. As described in the art, a complete intracellular signaling domain or a truncated portion of an intracellular signaling domain can be used. As will be understood by the skilled person, an effective truncated portion must transduces the effector function signal in substantially the same manner as the complete domain. Herein, the term "intracellular signaling domain" is meant to include any truncated portion of the intracellular signaling domain sufficient to transducing effector function signal.

In preferred embodiments of the invention, the CAR has a hinge region IgG1-CH2-CH3 (a first part of IgG1 is localized in the membrane and a second part of IgG1 is localized extracellularly outside the cell) and CD28 as transmembrane domain (a first part of CD28 is localized in the membrane and a second part of CD28 is localized intracellularly in the cytoplasm). CD8α may also be used as transmembrane domain and hinge region. Sequences derived from IgG4 may also be used.

As known from the art, signals generated through the T-Cell receptor (TCR) alone are insufficient for full activation of the T cell and that a secondary or co-stimulatory signal is also required. Thus, T cell activation can be said to be mediated by two distinct classes of intracellular signaling domains: primary signaling domains that initiate antigen-dependent primary activation through the TCR (e.g. a TCR/CD3 complex) and co-stimulatory signaling domains that act in an antigen-independent manner to provide a secondary or any co-stimulatory signal. In preferred embodiments, the CAR of the invention comprises an intracellular signaling domain that comprises one or more "co-stimulatory signaling domain". Consequently, in other preferred embodiments, isolated nucleic acid molecules of the invention encode an intracellular signaling domain comprising at least one costimulatory domain, so that the resulting intracellular signaling domain therefore comprises at least one costimulatory domain.

As used herein, the term "co-stimulatory signaling domain" or "co-stimulatory domain", refers to an intracellular signaling domain of a co-stimulatory molecule. Co-stimulatory molecules are cell surface molecules other than antigen receptors or Fc receptors that provide a second signal required for efficient T cells activation/function when binding to the antigen.

In the present description, the terms "polypeptide", "polypeptide fragment", "peptide" and "protein" are used interchangeably and have, unless specified to the contrary, the conventional meaning, i.e. a sequence of amino acids. Polypeptides are not limited to a specific length and may comprise a full length protein sequence or a fragment thereof. The polymer can be linear or branched, it can comprise modified amino acids, and it can be interrupted by non-amino acids. Further, polypeptides may have post-translational modifications, e.g. disulfide bond formation, glycosylation, lipidation, acetylation, phosphorylation, or any other manipulation or modification, such as conjugation with a labeling component, and more generally any modification occurring naturally occurring and/or non-naturally. Given the fact that the polypeptides of this invention are based on antibodies, in some embodiments, the polypeptides can occur as single chains or associated chains. Polypeptides can be prepared using any technique known in the art, i.e. recombinant and/or synthetic techniques. More particularly, polypeptides described herein encompass the CARs of the present disclosure, or sequences that have deletions from, additions to, and/or substitutions of one or more amino acid of a CAR as disclosed herein.

An "isolated peptide" or an "isolated polypeptide" and the like, as used herein, refer to in vitro isolation and/or purification of a peptide or polypeptide molecule from a cellular environment, and from association with other components of the cell. Similarly, an "isolated cell" refers to a cell that has been obtained from an in vivo tissue or organ and is substantially free of extracellular matrix.

The term "vector" is used herein to refer to a nucleic acid molecule capable transferring or transporting another nucleic acid molecule. More generally, a "vector" means a construct, which is capable of delivering, and expressing, one or more gene(s) or sequence(s) of interest in a host cell. The nucleic acid that is to be transported or transferred is linked to the vector nucleic acid molecule in any way known in the art, e.g. by insertion. A vector may include sequences that direct autonomous replication in a cell, or may include sequences sufficient to allow integration into host cell DNA. The present invention also provides a vector comprising a nucleic acid molecule encoding the CAR of the invention. The vector may be selected from a DNA, a RNA, phage vector, a plasmid, a lentivirus vector, an adenoviral vector, or a retrovirus vector. The vector of the invention preferably comprises a promoter, such as an EF-1 alpha promoter. The term "promoter" as used herein refers to a recognition site of a polynucleotide (DNA or RNA) to which an RNA polymerase binds. As known by the skilled person, RNA polymerase initiates the transcription of the polynucleotides operably linked to the promoter. In a particular embodiment, it may be desirable to express a polynucleotide comprising a CAR from a promoter that provides stable and long-term CAR expression in T cells. A CAR expression under the control of a promoter further ensures sufficient expression levels to direct the T cells against cells expressing the target antigen.

Retroviruses are a common tool for gene delivery. In some embodiments, a retrovirus is used to deliver a polynucleotide encoding a chimeric antigen receptor (CAR) to a cell, preferentially an immune cell. As used herein, the term "retrovirus" refers to an RNA virus that reverse transcribes its genomic RNA into a linear double-stranded DNA copy and subsequently covalently integrates its genomic DNA into a host genome. Once the virus is integrated into the host genome, it is referred to as a "provirus". The provirus serves as a template for RNA polymerase and directs the expression of RNA molecules which encode the structural proteins and enzymes needed to produce new viral particles. As a consequence, T cells transduced with a vector of the invention can generate a stable, long-term, and persistent CAR-mediated T-cell response. In particular embodiments, the T cell is transduced with a lentiviral vector, i.e. lentivirus, encoding a CAR according to the present invention. The term "lentiviral vector" refers to a viral vector or plasmid containing structural and functional genetic elements, or portions thereof, including long terminal repeats (LTRs) that are primarily derived from a lentivirus. The term "lentivirus" refers to a group (or genus) of complex retroviruses. Well known lentiviruses include human immunodeficiency virus (HIV, e.g. type 1 or type 2); visna-maedi virus (VMV); the caprine arthritis-encephalitis virus (CAEV); equine infectious anemia virus (EIAV); feline immunodeficiency virus (FIV); bovine immune deficiency virus (BIV); and simian immunodeficiency virus (SIV). The term "self-inactivating" (SIN) vectors refers to replication-defective vectors, e.g. retroviral or lentiviral vectors, in which the 3' LTR enhancer-promoter region, known as the U3 region, has been modified (for instance by deletion or substitution) to prevent viral transcription beyond the first round of viral replication.

The term "purified" and "isolated" it is meant, when referring to a molecule, such as a polypeptide or an antibody or a nucleotide sequence of the invention, that the indicated molecule is present in the absence of other biological macromolecules of the same type. More specifically the term "purified" as used herein in particular means at least 85%, 90%, 95% or 98% by weight, of biological macromolecules of the same type are present. More specifically the term "isolated" nucleic acid molecule that encodes a particular polypeptide refers to a nucleic acid molecule that is substantially free of other nucleic acid molecules that do not encode the subject polypeptide; however, the molecule may include some additional bases or moieties, which do not deleteriously affect the basic characteristics of the composition.

The term "antigen", "target" or "target antigen" refers to a molecule or a portion of a molecule that is capable of being bound by an antibody or an antibody-like binding protein. The term further refers to a molecule or a portion of a molecule that is capable of being used in an animal to produce antibodies that are capable of binding to an epitope of that antigen. A target antigen may have one or more epitopes. With respect to each target antigen recognized by an antibody or by an antibody-like binding protein, the antibody-like binding protein is capable of competing with an intact antibody that recognizes the target antigen.

The terms "CD123", "IL-3Ra" or "IL-3Rα", "Interleukine-3 Receptor alpha" or "Interleukine-3 Receptor a", are used interchangeably herein and refer to any native (human) IL-3Ra or CD123, unless otherwise indicated. The CD123 protein is an interleukin 3-specific subunit of a heterodimeric cytokine receptor (i.e. IL-3 Receptor or IL-3R). The IL-3R is comprised of a ligand specific alpha subunit, and a signal transducing common beta subunit (also known as CD131) shared by the receptors for interleukin 3 (IL3), colony stimulating factor 2 (CSF2 / GM-CSF), and interleukin 5 (IL5). The binding of CD123/IL-3Ra to IL3 depends on the beta subunit. The beta subunit is activated by the ligand binding, and is required for the biological activities of IL3. All of these foregoing terms for CD123 can refer to either a protein or nucleic acid sequence as indicated herein. The term "CD123/IL-3Ra" encompasses "full-length" unprocessed CD123/IL-3Ra, as well as any form of CD123/IL-3Ra that results from processing within the cell. The term also encompasses naturally occurring variants of CD123/IL-3Ra protein or nucleic acid, e.g. splice variants, allelic variants and isoforms. The CD123/IL-3Ra polypeptides and polynucleotides described herein can be isolated from a variety of sources, such as from human tissue types or from another source, or prepared by recombinant or synthetic methods. Examples of CD123/IL-3Ra sequences include, but are not limited to NCBI reference numbers NP_002174 & NM_002183 (protein and nucleic acid sequences for human CD123 variant 1), and NP_001254642 & NM_001267713 (protein and nucleic acid sequences for human CD 123 variant 2).

The term "anti-CD123 antibody", "anti-IL-3Ra antibody" or "anti-IL-3Rα antibody", "an antibody that specifically binds to CD123" or "an antibody that specifically binds to IL-3Ra/IL-3Rα refers to an antibody that is capable of binding CD123 with sufficient affinity such that the antibody is useful as a diagnostic and/or therapeutic agent in targeting CD123. Unless otherwise specified, the extent of binding of an anti-CD 123 antibody to an unrelated, non-CD123 protein is less than about 10% of the binding of the antibody to CD123 measured. More generally, the term "specifically binds" designates that an antibody binds to an epitope via its antigen-binding domain, and that the binding entails some complementarity between the antigen-binding domain and the epitope. According to this definition, an antibody is said to "specifically bind" to an epitope when it binds to that epitope, via its antigen-binding domain more readily than it would bind to a random, unrelated epitope. The term "specificity" is used herein to qualify the relative affinity by which a certain antibody binds to a certain epitope. For example, antibody "A" may be deemed to have a higher specificity for a given epitope than antibody "B" or antibody "A" may be said to bind to epitope "C" with a higher specificity than it has for related epitope "D." Accordingly, an antibody or antigen-binding fragment of the invention specifically binds to a CD123 antigen, in that it has a higher binding specificity to the CD123 antigen (from any species) than that to a non-CD123 antigen. More particularly, an antibody or antigen-binding fragment of the invention specifically binds to a human CD 123 antigen, in that it has a higher binding specificity to the human CD123 antigen than that to a non-human CD123 antigen (e.g. a mouse or a rat CD123). Occasionally, the term "preferentially binds" designates that an antibody specifically binds to an epitope more readily than it would bind to a related, similar, homologous, or analogous epitope. Hence, an antibody which preferentially binds to a given epitope would more likely bind to that epitope than to a related epitope, even though such an antibody may cross-react with the related epitope. For example, an antibody or antigen-binding fragment of the invention may preferentially bind to a human CD123 antigen over a mouse CD123.

According to the above, terms related to an antibody, a CAR, a vector or a cell of the invention are easily derived. Indeed, the general context of the description does not always require to stick exact same wording as labeled above. Some deviation may occur. For example, when referring to a CAR of a T cell it is not always required to specify that the CAR comprises a part directed to a specific antigen. i.e. "anti-something". Accordingly, and on a more general note, a CAR of the present invention that comprises a part that specifically binds to CD123 may be referred to not only as anti-CD123 CAR but also as CD123 CAR. In the same manner, a T cell that is transduced with a CAR that specifically binds to CD123 according to the invention, may be referred to as CD123 CAR T cell.

A "treatment" as used herein includes any beneficial or desirable effect on the symptoms or pathology of a disease or pathological condition, and may include any reductions in one or more measurable markers of the disease or condition being treated, e.g. auto-immune diseases. Treatment may comprise either the reduction of symptoms or any improvement regarding the symptoms a disease or condition. A treatment further may delay the progression of the disease or condition. Hence the term "treatment" does not necessarily means a complete eradication or cure of the disease or condition, or the associated symptoms thereof. With this in mind, the present invention disclosure provides for the treatment or prevention of BPDCN comprising administering to a subject in need thereof, a therapeutically effective amount of the T cells of the invention.

In this context, The T cells described herein may be administered either alone, or as a pharmaceutical composition (also called pharmaceutical formulation). The term "pharmaceutical composition" or "pharmaceutical formulation" refers to a preparation which is in such form as to permit the biological activity of the active ingredient to be effective, and which contains no additional components which are unacceptably toxic to a subject to which the composition/formulation would be administered. Such formulation can be sterile. Pharmaceutical compositions may comprise T cells according to the invention alone, or in combination with one or more pharmaceutically or physiologically acceptable carriers, diluents or excipients. Such compositions may comprise buffers such as neutral buffered saline, phosphate buffered saline and the like; carbohydrates such as glucose, mannose, sucrose or dextran, mannitol; proteins; polypeptides or amino acids such as glycine; antioxidants; chelating agents such as EDTA or glutathione; adjuvants, and stabilizers. The terms "pharmaceutically acceptable" or "physiologically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio. Compositions of the present invention are preferably formulated for parenteral administration. A "parenteral administration" may refer to administration modes other than enteral and topical administration. Usually parenteral administration is done by injection, such as intravascular, intravenous, intramuscular, intraarterial, intrathecal, intra-capsular, intra-orbital, intra-tumoral, intra-cardiac, intradermal, intraperitoneal, transtracheal, subcutaneous, subcuticular, intraarticular, subcapsular, subarachnoid, intraspinal and intrasternal injection and/or infusion. Preferentially, the CAR-modified T cells or the compositions of the invention are administered to a subject by direct injection into a site of inflammation, lymph node, systemic circulation, or site of infection. Generally, the invention is useful to treat a subject diagnosed with a disease overexpressing CD123, particularly patients having BPDCN. The treatment comprises removing immune effector cells from the subject, genetically modifying said immune effector cells with a vector comprising a nucleic acid encoding a CAR as contemplated herein, thereby producing a population of modified immune effector cells, and administering the population of modified immune effector cells to the same subject. In a preferred embodiment, the immune effector cells are T cells. The quantity, frequency of the administration are determined with regard to the patient physical state (condition of the patient, the type and severity of the patient's disease). Appropriate dosages may also be determined with the help of animal models followed by clinical trials. When referring to dosages, the term "effective amount" is an amount sufficient to carry out a specifically stated purpose. The so called "therapeutically effective amount" of an active ingredient, such as a genetically modified T cell, may depend on factors such as the disease and its state, the age, sex, and weight of the patient, and further depend on the ability of the cells to induce the desired response in the patient. A therapeutically effective amount is also one in which any toxic or detrimental effects of the virus or transduced therapeutic cells are outweighed by the therapeutically beneficial effects. It can generally be stated that a pharmaceutical composition comprising the T cells described herein may be administered at a dosage of 10⁴ to 10⁹ cells/kg body weight, preferably 10⁵ to 10⁶ cells/kg body weight, including all integer values within those ranges. More generally, the term "therapeutically effective amount" refers to an amount of an antibody, active ingredient or other drug effective for treating a disease or disorder in a subject.

In the case of auto-immune diseases, the therapeutically effective amount of the drug can reduce the number of cells implicated in physiopath of the disease and consequently the quantity/ concentration of inflammatory molecules; reduce the inflammation size inhibit the disease (i.e. slow down to some extent or stop the disease); relieve to some extent one or more of the symptoms associated with the auto-immune disease and/or result in a favorable response such as increased progression-free survival (PFS), disease-free survival (DFS), or overall survival (OS), complete response (CR), partial response (PR), or, in some cases, stable disease (SD), a decrease in progressive disease (PD), a reduced time to progression (TTP), or any combination thereof. A "prophylactically effective amount" refers to an amount effective, at dosages and for periods of time necessary, to achieve the desired prophylactic result. Usually, the prophylactically effective amount is less than the therapeutically effective amount since a prophylactic dose is used in subjects prior to or at an earlier stage of disease.

In particular embodiments, prior to *in vitro* manipulation or genetic modification of the immune effector cells described herein, the source of cells is obtained from a subject. In particular embodiments, the immune effector cells expressing the CAR of the invention at its membrane comprise T cells. T cells can be obtained from a number of sources including, but not limited to, peripheral blood mononuclear cells, bone marrow, lymph nodes tissue, cord blood, thymus issue, tissue from a site of infection, ascites, pleural effusion, spleen tissue. In certain embodiments, T cells can be obtained from a unit of blood collected from a subject using any number of techniques known to the skilled person, such as FICOLL^{™} separation. In another embodiment, cells from the circulating blood of an individual are obtained by apheresis. The apheresis product typically contains lymphocytes, including T cells, monocytes, granulocyte, B cells, other nucleated white blood cells, red blood cells, and platelets. In one embodiment, the cells collected by apheresis may be washed to remove the plasma fraction and to place the cells in an appropriate buffer or media for subsequent processing. In other embodiments, T cells are isolated from peripheral blood mononuclear cells by lysing the red blood cells and depleting the monocytes, for example, by centrifugation through a PERCOLL^{™} gradient. A specific subpopulation of T cells, expressing one or several markers like CD3, CD4 or CD8 can be further isolated by positive or negative selection techniques. For example, enrichment of a T cell population by negative selection can be accomplished with a combination of antibodies directed to surface markers not expressed on the negatively selected cells.

In a more general context, the present description highlights a cellular therapy where T cells are genetically engineered (or genetically modified) *ex-vivo* to express a CAR and the CAR T cell is infused to a patient, preferably a human, in need thereof.

The infused cell is able to kill cells in the patient thought expression of specific molecules (e.g. CD123). As explained above, and unlike antibody therapies, T cells comprising a CAR are able to replicate *in vivo* resulting in long-term persistence that can lead to sustained control of auto-immune diseases. Moreover, CARs allow for the redirection and activation of effector T cells towards any cell surface molecule upon binding by the antibody derived receptor, and are independent of MHC restriction (MHC-restricted antigen recognition; MHC stands for Major Histocompatibility Complex). As mentioned above, genetically engineered T cells of the invention are constructed starting from T cells collected from the patient himself/herself (autologous), but they can also originate from other allogenic donors to provide allogenic genetically-engineered T cells in bone marrow or peripheral hematopoietic stem cell allograft context (Donor lymphocytes infusion). These T cells expressing a CAR molecule according to the invention are useful to treat an auto-immune disease in a mammal, preferably a human.

The invention is further described in detail with reference to experimental data and specific examples. The examples are provided for purposes of illustration only, and are not intended to be limiting unless otherwise specified. While the following detailed description concentrates on

The invention can generally provide improvement and/or solutions for auto-immune diseases wherein a CD123 overexpression occurs.

Thus, a major focus of the present invention is to provide a treatment for auto-immune diseases, and specifically for Cutaneous Lupus Erythemathosus, Dermatomyositis, Psoriasis and systemic sclerosis.

However, developing a treatment or drug is accompanied by hurdles for the skilled person. For instance, a drug might turn out to be too toxic for the treatment of non-deadly diseases. Further the development is often extremely costly which generally turns away a skilled person from diseases for which only a small marked is available.

Moreover, treatments such as those implicating CARs are difficult to develop. For instance, constructing CARs is an extremely difficult task. This is often due to the fact that the CARs are constructed from patients' cells, or at least by using specific cells extracted from a patient such as T Lymphocytes. Yet those cells are often unnormal in patients affected by autoimmune diseases. This is particularly the case for T Lymphocytes in autoimmune diseases since the cells are autoimmune themselves.

Numerous other hurdles need to be overcome in order to consider CAR treatments. Another example is that in some diseases the CARs need to diffuse into the skin, which can represent some additional hurdles.

According to an embodiment of the present invention, a CAR has been genetically engineered to be made of a single chain variable fragment (ScFv) of heavy and light chains of a monoclonal antibody (MAb) linked to the intracellular signaling chain of a T cell receptor (TCR). Preferably, the intracellular part the CAR comprises a CD3ζ and costimulatory domains such as CD28 and 4-1BB that enable better activation and cell signaling. The CAR can be qualified as a third generation CAR. For more details on CAR: see the review of Andrew D. Fesnak, Carl H. June and Bruce L. Levine, Engineered T cells: the promise and challenges of cancer immunotherapy, Nature, vol. 16, sept. 2016. The ScFV allows the recognition of an antigen independently of major histocompatibility complex (MHC) presentation.

Growing clinical trials show that patients under CAR therapy develop toxicity. This is a major problem in the development of efficient CARs. The Applicant developed a specific CAR that overcomes the problems of the prior art (cf. EP3753954). More particularly, the Applicant engineered both third generation retroviral and lentiviral CARs. The CARs of the invention overcome the problems of the prior art, and particularly those related to cytotoxicity. The CARs of the invention have improved functional activity on BPDCN cells (BPDCN cells lines, PDX cells and primary BPDCN cells) *in vitro* as *in vivo* in several mice models of BPDCN. The evaluation of their potential cytotoxicity on CD123^{low} positive cells are surprisingly promising.

According to the invention, monoclonal antibodies (Mab) directed against human CD123 were produced. The monoclonal antibodies used with the invention can be used in both allogenic and autologous context.

The production method of the Anti-CD123 monoclonal antibodies included immunization of mouse with recombinant CD123 protein. More specifically, five mice were immunized with the recombinant CD123 available under the reference #301-R3-025, available at R&D Systems, Minnesota, USA). Immunization was carried out by footpad and/or intraperitoneal administration. Lymph-nodes and/or splenic B cells were used to produce hybridomas secreting Mab. Hybridomas were selected based on the affinity and specificity of MAbs using CD123⁺ and CD123⁻ cell lines.

For the invention a specific antibody was selected. Herein the antibody is named 18B4D5 (also referred herein as AB1, or sometimes abbreviated as B4D5). Molecular characterization and DNA sequencing has been made according to SANGER method.

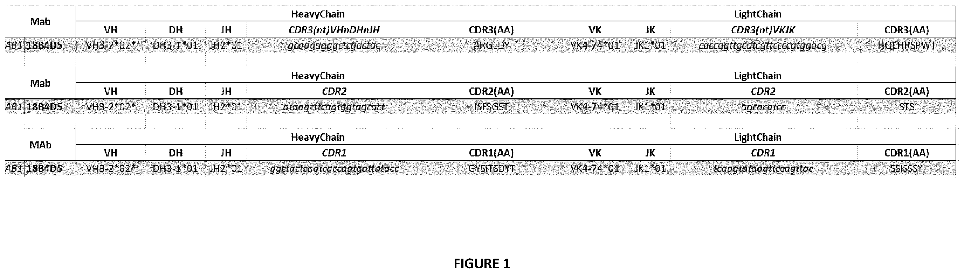

VDJ and VJ gene rearrangements were sequenced and identified after alignment of consensus nucleotide sequences against the IMGT^{®} database using the VQUEST online tool; cf. Brochet X, Lefranc MP, Giudicelli V. IMGT/V-QUEST: the highly customized and integrated system for IG and TR standardized V-J and V-D-J sequence analysis, Nucleic acids research, 2008; 36(Web Server issue):W503-8. Sequences (nucleotide sequences and amino acid sequences) of the antibody 18B4D5 are listed in figures 1 to 5.

Figure 1 shows the nucleotide sequence and the amino acid sequence of complementary determining region 1 (CDR1), complementary determining region 2 (CDR2) and complementary determining region 3 (CDR3) of both the heavy chain and the light chain of the antibody 18B4D5 used in the invention.

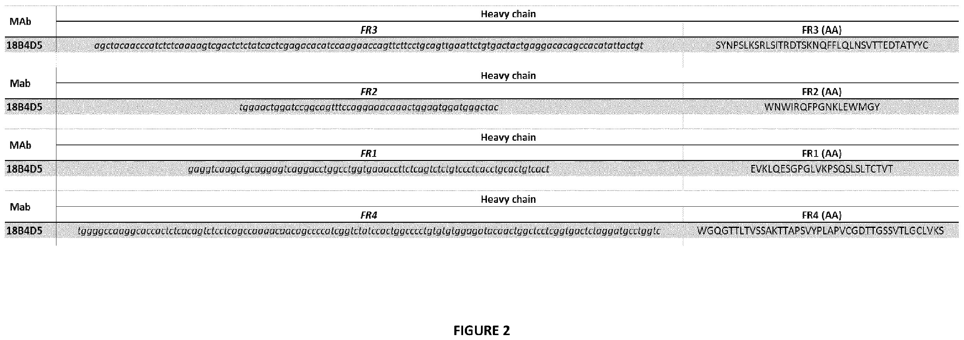

Figure 2 shows the nucleotide sequence and the amino acid sequence of framework region 1 (FR1), framework region 2 (FR2), framework region 3 (FR3) and framework region 4 (FR4) of the heavy chain of the antibody 18B4D5 used in the invention.

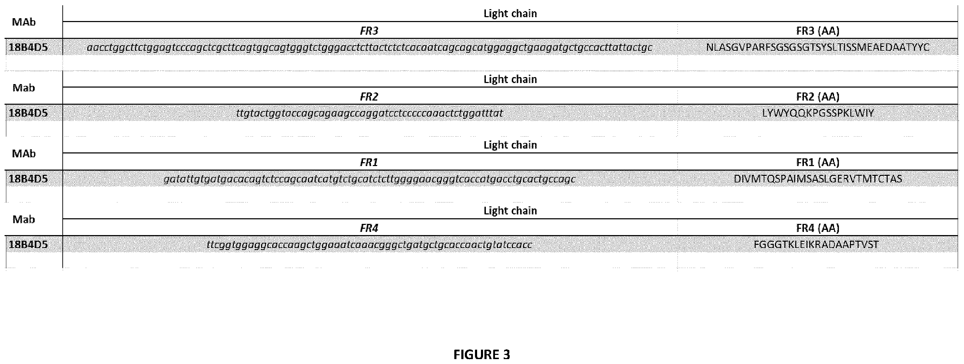

Figure 3 shows the nucleotide sequence and the amino acid sequence of framework region 1 (FR1), framework region 2 (FR2), framework region 3 (FR3) and framework region 4 (FR4) of the light chain of the antibody 18B4D5 used in the invention.

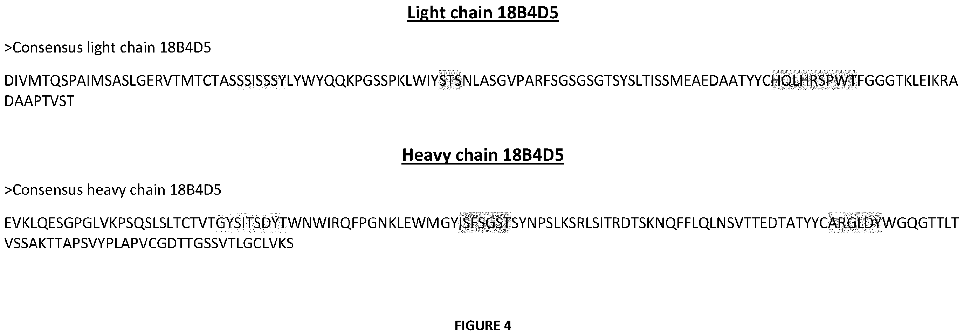

Figure 4 shows the consensus amino acid sequence of the light chain and the consensus amino acid sequence of the heavy chain of the antibody AB1 used with the invention named 18B4D5 or B4D5. Amino acids corresponding to the CDR1, CDR2 and CDR3 are respectively highlighted from left to right in reading order.

The sequence homology, using the VQUEST online tool as described above, for heavy chain VH and JH as well as for light chain VK and JK of the antibody used in the invention is shown in table 1.

**Table 1: Sequence homology of variable chains.**

| **Antibody** | | **Heavy Chain** | | | **Light Chain** | | |
|---|---|---|---|---|---|---|---|
| | | | | **Homologie** | | | **homologie** |
| ***AB1*** | **18B4D5** | **VH** | VH 3-2*02* | 97,22 | **VK** | VK 4-74*01 | 96,1 |
| | | **JH** | JH2*01 | 89,13 | **JK** | JK 1*01 | 100 |

By selecting this specific antibody the Applicant designed a specific Chimeric Antigen Receptor T cell (CAR-T) that is highly effective in treating auto-immune diseases.

In order to do so the inventors initially based their research on knowledge regarding CAR-T therapy. CAR-T therapy is one of the most promising cell-based therapies with more than 800 clinical studies conducted worldwide to evaluate numerous targets and indications, cf. Singh, A. K. & McGuirk, J. P. CAR T cells: continuation in a revolution of immunotherapy - Lancet Oncol. 21, e168-e178 (2020). It is mainly developed in the field of oncology, and in particular in hematology. It provides promising results enabling a high remission rate in patients that are in relapse or are refractory to other treatments Maude, cf. S. L. et al., Tisagenlecleucel in Children and Young Adults with B-Cell Lymphoblastic Leukemia - N. Engl. J. Med. 378, 439-448 (2018).

More recently, use of CAR-T therapy has been extended to other fields such as infectiology, cardiac fibrosis or autoimmune diseases (AID), cf. Aghajanian, H., Rurik, J. G. & Epstein, J. A. CAR-based therapies: opportunities for immuno-medicine beyond cancer - Nat. Metab. 4, 163-169 (2022). Also, a report on rapid and durable remission in a patient with severe lupus treated with a CD19-targeted CAR-T has been recently made, cf. Mougiakakos, D. et al., CD19-Targeted CAR T Cells in Refractory Systemic Lupus Erythematosus - N. Engl. J. Med. 385, 567-569 (2021).

The Applicant thus concentrated on the use of specific autologous CAR-T's in autoimmune diseases. However, a major difficulty is linked to the identification of an effective CAR-T that also shows cytotoxicity compatible with patient treatment. Such an identification is, among other difficulties, particularly challenging due to numerous CAR-T available in the art.

Another study showed that other patients have benefited from the anti-CD19 CAR-T for their refractory lupus and have obtained satisfying clinical results, cf. Mackensen, A. et al., Anti-CD19 CAR T cell therapy for refractory systemic lupus erythematosus - Nat. Med. 28, 2124-2132 (2022). The injected CAR-T's did not seem present any major adverse effects on the patients. Various pre-clinical studies are currently being made in the field of anti-CD19 CAR-T. Some CAR-T showed preclinical efficacy in autoimmune diseases and are in clinical trials today: anti-CD19 (NCT03030976, in lupus), anti-CD19/BCMA (NCT05030779 in lupus), anti-BCMA (NCT0414051 in generalized myasthenia gravis) and an anti DSG34.6 CAAR-T (NCT04422912 in Pemphigus Vulgaris) Ellebrecht, C. T. et al., Reengineering chimeric antigen receptor T cells for targeted therapy of autoimmune disease - Science 353, 179-184 (2016).

The Applicant oriented his research away from the above CAR-T in order to identify a new CART-T generation active against auto-immune diseases. By doing so the Applicant managed to identify the feasibility of CAR-T transfer in general, and further to identify a CAR-T that is not only tolerable for the patient, but also highly effective in systemic lupus erythematosus.

Yet, the development of a CAR-T therapy highly depends on the choice of its antigenic target. Thus a challenging aspect is that the target must be as specific as possible to the cell to be eliminated, in order to reduce the so called "off target off tumor" adverse effects.

The Applicant discovered, not without surprise, that an anti-CD123 CAR-T meets the above challenges and requirements.

The CD123 marker is the subunit α of the IL-3 heterodimer receptor. It is strongly expressed in pDCs and basophilic cells and weakly expressed in other cell types such as monocytes, myeloid progenitors and endothelial cells. IL-3 signaling is essential for the survival of pDCs, cf. Bôle-Richard, E. et al., CD28/4-1BB CD123 CAR T cells in blastic plasmacytoid dendritic cell neoplasm - Leukemia 34, 3228-3241 (2020) and Oon, S. et al., A cytotoxic anti-IL-3Rα antibody targets key cells and cytokines implicated in systemic lupus erythematosus - JCI Insight 1, (2016).

The Applicant considered that pDCs represent between 0.1-0.5% of circulating mononuclear cells, and further, that pDCs are part of the innate immune system, cf. Bode, C. et al., Human plasmacytoid dentritic cells elicit a Type I Interferon response by sensing DNA via the cGAS-STING signaling pathway - Eur. J. Immunol. 46, 1615-1621 (2016). Moreover, the Applicant considered that pDCs have a rather high capacity to secrete type I interferon (IFN-I). IFN-I is a major cytokine of innate immune system which is produced via the stimulation of TLR7 and TLR9. TLR7 and TLR9 are cytosolic receptors of pDCs that are mainly activated by RNA and DNA fragments, cf. Ah Kioon, M. D. et al., Plasmacytoid dendritic cells promote systemic sclerosis with a key role for TLR8 - Sci. Transl. Med. 10, eaam8458 (2018); Kafaja, S. et al., pDCs in lung and skin fibrosis in a bleomycin-induced model and patients with systemic sclerosis - JCI Insight 3, 98380 (2018); and Ganguly, D. et al., Self-RNA-antimicrobial peptide complexes activate human dendritic cells through TLR7 and TLR8. J. Exp. Med. 206, 1983-1994 (2009).

Some studies suggest that pDCs might be involved in the development of autoimmune diseases and/or autoinflammatory diseases related to overproduction of IFN-I, cf. Li, S., Wu, J., Zhu, S., Liu, Y.-J. & Chen, J. Disease-Associated Plasmacytoid Dendritic Cells. Front. Immunol. 8, 1268 (2017).

The Applicant also considered that pDCs have a function of antigen presentation and a function of secretion of pro-inflammatory cytokines (IFN-I, IL-6, IL-12, CXCL8, CXCL10, CCl3 and CCL4). Further, the Applicants research was oriented towards data showing that patients with specific autoimmune diseases have a lower circulating pDCs rate than healthy persons. However, in these patients the pDCs infiltrate injured and inflammatory tissues. Consequently pDCs seem to have a strong capacity for tissue infiltration, particularly in the skin in systemic scleroderma (SSc) or in the kidney in lupus erythematosus, cf. Kokaji, A. I., Holland, S., Fairhurst, M. A., Thomas, T. E. & Guilbault, B. G. A simple one-step method for isolating highly purified plasmacytoid dendritic cells from human peripheral blood (78.33) - J. Immunol. 182, 78.33-78.33 (2009); Vermi, W. et al., Cutaneous distribution of plasmacytoid dendritic cells in lupus erythematosus. Selective tropism at the site of epithelial apoptotic damage - Immunobiology 214, 877-886 (2009); Albanesi, C., Scarponi, C., Bosisio, D., Sozzani, S. & Girolomoni, G. Immune functions and recruitment of plasmacytoid dendritic cells in psoriasis. Autoimmunity 43, 215-219 (2010); Nestle, F. O. et al., Plasmacytoid predendritic cells initiate psoriasis through interferon-alpha production. J. Exp. Med. 202, 135-143 (2005); and Bell, E. Plasmacytoid dendritic cells in psoriasis. Nat. Rev. Immunol. 7, 839-839 (2007).

Other autoimmune diseases seem related to pDCs activity that produces 1'IFN-I, such as psoriasis and dermatomyositis, cf. Albanesi, C. et al., Chemerin expression marks early psoriatic skin lesions and correlates with plasmacytoid dendritic cell recruitment - J. Exp. Med. 206, 249-258 (2009); and Tezak, Z. et al., Gene expression profiling in DQA1*0501+ children with untreated dermatomyositis: A novel model of pathogenesis - J. Immunol. 168, 4154-4163 (2002).

The infiltration of pDCs in the lung in SSc, with IFN-α and CXCL4 secretion in humans and mouse is considered a poor prognostic, associated with skin and lung fibrosis and a more severe clinical profile, cf. van Bon, L. et al., Proteome-wide analysis and CXCL4 as a biomarker in systemic sclerosis - N. Engl. J. Med. 370, 433-443 (2014). Said otherwise, the high level of cytokine/chimiokine (IFN-α and CXCL4) identified in patients is associated with skin and lung fibrosis and a more severe clinical profiles. This shows that the pDCs that secrete these cytokines play an unfavorable role in this pathology - This is also observed in mouse models. As a consequence, pDC infiltration is a poor prognostic factor.

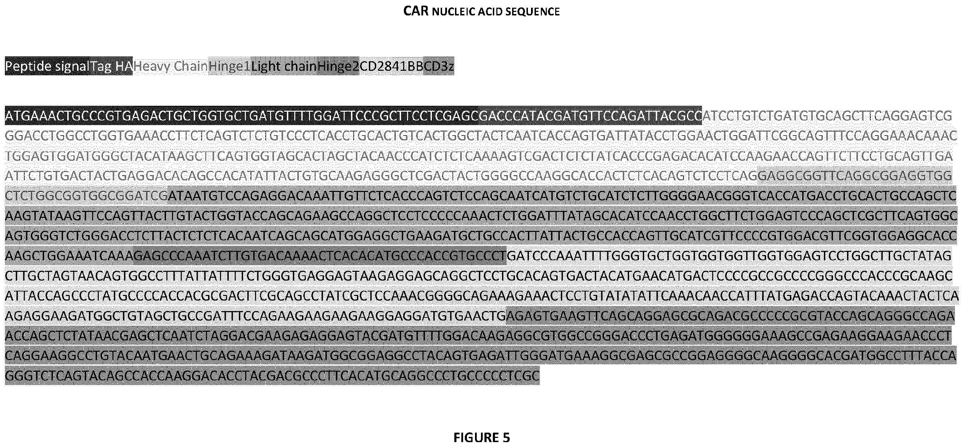

Figure 5 shows the nucleic acid sequence of a CAR according to the invention.

The CAR is built with antibody AB1. Nucleic acids corresponding to the Peptide signal, Tag HA, Heavy Chain, Hinge1, Light chain, Hinge2, CD28, 4.1BB, and CD3z are respectively highlighted in different shades from left to right in reading order. Further details for the construction of the CAR123 of the invention is disclosed in EP3753954 to which the reader may refer.

Accordingly, an object of the present invention is an isolated nucleic acid molecule encoding a chimeric antigen receptor (CAR), wherein the nucleic acid molecule consists of a sequence having an identity of at least 85%, preferentially 90%, more preferentially 95% to SEQ ID NO : 6 for use in a treatment of autoimmune diseases.

Another object of the present invention is an isolated nucleic acid molecule encoding a chimeric antigen receptor (CAR), wherein the nucleic acid consists of SEQ ID NO : 6 for use in a treatment of autoimmune diseases.

Affinity of the antibody to CD123 is tested on streptavidin biosensors. Herein, to determine K_{D} values seven different concentrations of CD123 were used on immobilized antibodies of the invention. The results were analyzed according to the Octet system available at FortéBio.

Results of antibody affinity is shown in table 2.

**Table 2: Antibody affinity.**

| **Loading Sample ID** | **Conc. (nM)** | **Response** | **KD (M)** | **kon (1/Ms)** | **Kdis (1/s)** | **Full R^2** |
|---|---|---|---|---|---|---|
| AB1 (18B4D5) | 50 | 0,6972 | <1.0E-12 | 1,22E+05 | <1.0E-07 | 0,9988 |
| AB1 (18B4D5) | 25 | 0,4202 | <1.0E-12 | 1,22E+05 | <1.0E-07 | 0,9988 |
| AB1 (18B4D5) | 12,5 | 0,2301 | <1.0E-12 | 1,22E+05 | <1.0E-07 | 0,9988 |
| AB1 (18B4D5) | 6,25 | 0, 12 | <1.0E-12 | 1,22E+05 | <1.0E-07 | 0,9988 |

The Applicant isolated mononuclear cells by means of Ficoll. T lymphocytes were isolated from the mononuclear cells and then activated by magnetic sorting (anti-CD3/CD28 magnetic beads, Gibco^{™}) . Activated T lymphocytes were cultured in RPMI 10% (supplemented with human serum) and IL-2 medium. The pDCs were isolated by negative sorting (EasySep^{™} Human pDC Isolation kit, StemCell) and phenotyped, both before and after sorting, to determine sorting purity before freezing at -80°C.

Two days after the activation of the T lymphocytes, they were transduced with a lentiviral vector. Transduction efficiency was evaluated after seven days post-transduction (CD3+/CD19+). Non-transduced T lymphocytes (C0) served as controls for all experiments.

The functionality of the CAR123 was studied after its co-culture with T lymphocytes (C0 or CAR123) for 6 hours with target cells (sorted pDCs or CAL-1). The percentage of cytotoxicity by flow cytometry was then assessed.

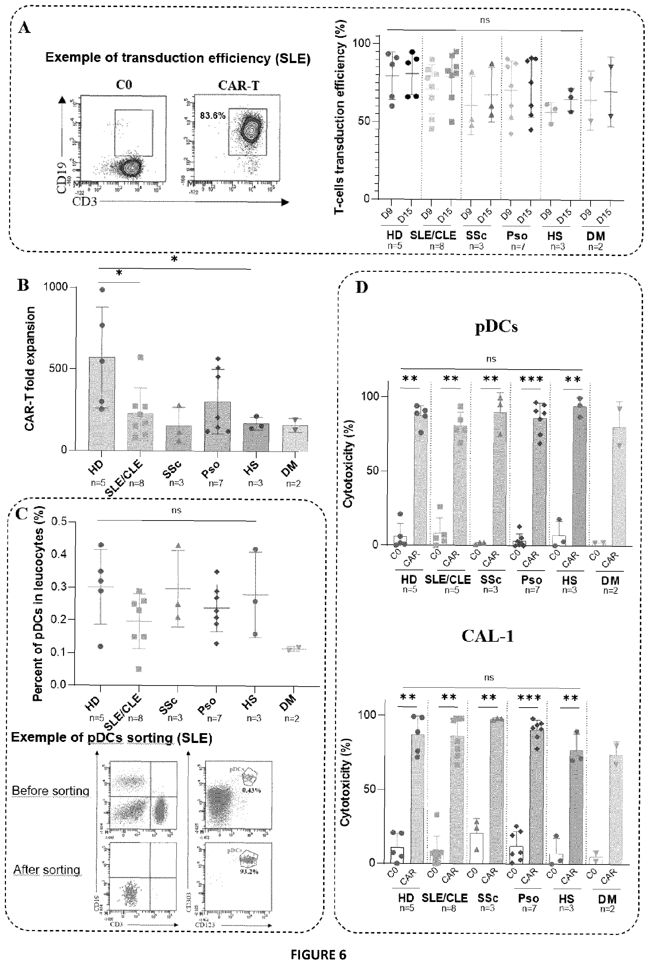

Figure 6 shows results of the above discussed analyses regarding the CAR123 of the invention using different blood samples: Healthy donor (HD), Patients with systemic lupus (SLE) or cutaneous-articular lupus (CLE), Patients with dermatomyositis (DM), Patients with psoriasis (PSO), and Patients with systemic scleroderma (SSc).

Figure 6A shows the T cells transduction efficiency which is determined by the expression of CD3+/CD19+ seven days after transduction. It corresponds to the percentage of T cells that have been transduced, i.e. expressing the CAR of the invention on the surface. No significant differences were osbserved between HD and patients.

Figure 6B shows T cells fold expansion that was determined after 9 days of culture in RPMI medium supplemented with human serum and IL-2. The results show that T cells from patients are able to activate and proliferate efficiently.

Figure 6C shows that the percentage of pDCs, determined by flow cytometry is identical when comparing HD with patients. pDcs express CD123 and BDCA2.

Figure 6D shows the ability to sort pDCs using the *EasyStem^{™} Human pDC Isolation Kit (StemCell).*

Figure 6E shows the percentage of the frequency of killed target cells. The percentage of cytotoxicity is assessed by the percentage of lysis target cells (pDCs or CAL-1) 6 hours after co-culture with effectors (C0 ou CAR-T) at an effector/target ratio of 5/1. CAL-1 are used as a positive control (CD123+ BPDCN cell line). We obtaina strong cytotoxicity with CAR-T produced from patients against CAL-1 or pDCs, with no diffrence with CAR-T produced from HD : these results show that we can obtain functional CAR-T from patients, , as well as from lymphocytes T coming from healthy pateints.

Regarding the Statistical test : a nonparametric t-test (Wilcoxon) was used for statistical analyses. Data are represented by the mean and standard deviation. p-value <0.05 is considered statistically significant, ***p<0.001.

The results show the strong capacity of the autologous CAR123 of the invention, that was produced from samples of patients with pDC-mediated autoimmune diseases, to eliminate circulating autologous pDCs *in vitro.* As known by the skilled person these results also apply to *in vivo* models.

Other cells express the CD123 molecule on their surface. For instance, this is the case of basophil polynuclear (high expression) and monocytes (lower expression). These cells are therefore also a target of the CAR123 of the invention. Consequently, the Applicant has studied the CAR123 of the invention with regard to said autoimmune diseases.

More particularity, in lupus, the Applicant has considered the important infiltration of monocytes and macrophages in the renal inflammatory lesions of patients with lupus nephropathy. Furthermore, the presence of monocytes (CD16+ subpopulations) in the lesions increases the activation of autoreactive lymphocytes T and lymphocytes B on one hand, increases antigenic presentation, and participates in the poor clearance of apoptotic bodies and immune complexes. Furthermore they secrete pro-inflammatory cytokines in large quantities; cf. Katsiari, C. G. et al., Aberrant expression of the costimulatory molecule CD40 ligand on monocytes from patients with systemic lupus erythematosus, Clin. Immunol. Orlando Fla 103, 54-62 (2002); Kwant, L. E. et al., Macrophages in Lupus Nephritis: Exploring a potential new therapeutic avenue, Autoimmun. Rev. 21, 103211 (2022); Miyagawa, F., Tagaya, Y., Ozato, K., Horie, K. & Asada, H. Inflammatory monocyte-derived dendritic cells mediate autoimmunity in murine model of systemic lupus erythematosus, J. Transl. Autoimmun. 3, 100060 (2020).

Regarding SSc, the Applicant has considered the importance of the implication of monocytes/macrophages. In fact, in SSc, the percentage of circulating monocytes is significantly higher compared to healthy donors, and the subpopulation of CD16-expressing monocytes is higher in diffuse SSc compared to limited SSc. This subpopulation is also associated with greater severity with skin fibrosis, lung fibrosis and pulmonary dysfunction suggesting a link between these monocytes and the pathogenesis of fibrosis in SSc; cf. Lescoat, A. et al., CD16-positive circulating monocytes and fibrotic manifestations of systemic sclerosis, Clin. Rheumatol. 36, 1649-1654 (2017); Laurent, P. et al., Innate Immunity in Systemic Sclerosis Fibrosis: Recent Advances, Front. Immunol. 9, (2018). Furthermore, the Applicant considered that profibrotic cells derived from circulating CD14+ monocytes in SSc are associated with interstitial lung disease and involved in the pathogenesis of SSc, and further the fact that there exists an association between the presence of monocytes and fibrotic manifestations with CD14+ monocyte/macrophage infiltration in lung tissue; cf. Higashi-Kuwata, N. et al., Characterization of monocyte/macrophage subsets in the skin and peripheral blood derived from patients with systemic sclerosis. Arthritis Res. Ther. 12, R128 (2010).

In lupus, the deleterious role of basophil polynuclear has been demonstrated. The presence of autoreactive IgE in the serum of lupus patients was found, and this presence was correlated with the clinical severity score with a higher rate in patients with lupus nephritis. basophil polynuclear have been shown to contribute to the maintenance of a pathogenic Th2 environment in lupus, leading to the maturation of autoreactive Lymphocytes B and their production of autoreactive IgE. Another study showed that basophil polynuclears and the Th2 environment were linked to the development of lupus nephritis; cf. Charles, N., Hardwick, D., Daugas, E., Illei, G. G. & Rivera, J., Basophils and the T helper 2 environment can promote the development of lupus nephritis, Nat. Med. 16, 701-707 (2010); Pellefigues, C. & Charles, N., The deleterious role of basophils in systemic lupus erythematosus, Curr. Opin. Immunol. 25, 10.1016/j.coi.2013.10.003 (2013). In SSc, peripheral basophil polynuclear are able to stimulate LBs and fibroblasts, thereby participating in fibrosis, suggesting an important role for basophil polynuclear in the pathophysiology of SSc; cf. Basophils Are Activated and Stimulate Both B Cells and Fibroblasts in Systemic Sclerosis. ACR Meeting Abstracts https://acrabstracts.org/abstract/basophils-are-activated-and-stimulate-both-b-cells-and-fibroblasts-in-systemic-sclerosis/.

The CAR123 of the invention has an effect on different immune cells, which is an advantage in the above discussed diseases which each involve different immune players in their pathogenesis.

## Claims

1. An isolated chimeric antigen receptor (CAR) molecule comprising an antibody or antibody fragment which includes an anti-CD123 binding domain, a transmembrane domain, and an intracellular signaling domain comprising at least a stimulatory domain, and wherein said anti-CD123 binding domain comprises a heavy chain comprising a complementary determining region 1 (CDR1) having at least 90% identity to amino acid sequence SEQ ID NO : 1, a complementary determining region 2 (CDR2) having at least 90% identity to amino acid sequence SEQ ID NO : 2, and a complementary determining region 3 (CDR3) having at least 90% identity to amino acid sequence SEQ ID NO : 3, and a light chain comprising a complementary determining region 1 (CDR1) having at least 90% identity to amino acid sequence SEQ ID NO : 4, a complementary determining region 2 (CDR2) having at least 90% identity to amino acid sequence Serine-Threonine-Serine (STS), and a complementary determining region 3 (CDR3) having at least 90% identity to amino acid sequence SEQ ID NO : 5 for use in the treatment of an autoimmune disease.

2. The isolated chimeric antigen receptor according to claim 1, wherein the autoimmune disease is selected from the group consisting of Cutaneous Lupus Erythemathosus, Dermatomyositis, Psoriasis and systemic sclerosis.

3. The isolated chimeric antigen receptor according to one of the preceding claims, wherein the anti-CD123 binding domain is selected from the group consisting of an antibody, a Fv, a scFv, a Fab, or another antibody fragment, preferably a scFv.

4. The isolated chimeric antigen receptor according to one of the preceding claims, wherein the intracellular signaling domain is CD3-zeta (CD3ζ), optionally comprising a costimulatory domain selected from the group consisting of CD28, 4.1BB, Inducible T-cell COStimulator (ICOS), OX-40 or a combination thereof.

5. The isolated chimeric antigen receptor of claim 1, having at least 90% identity, preferentially 100% identity, to nucleic acid sequence SEQ ID NO : 6.

6. An expression vector comprising a nucleic acid molecule as defined in claim 5, wherein the vector is selected from the group consisting of a DNA, a RNA, a plasmid, a lentivirus vector, an adenoviral vector, or a retrovirus vector for use in the treatment of an autoimmune disease.

7. The expression vector of claim 6, wherein said autoimmune disease is selected from the group consisting of Cutaneous Lupus Erythemathosus, Dermatomyositis, Psoriasis and systemic sclerosis.

8. An engineered immune cell comprising the nucleic acid molecule of claim 5 or the vector of claim 6 for use in the treatment of an autoimmune disease.

9. The engineered immune cell of claim 8, wherein said autoimmune disease is selected from the group consisting of Cutaneous Lupus Erythemathosus, Dermatomyositis, Psoriasis and systemic sclerosis.

10. A pharmaceutical composition comprising an isolated chimeric antigen receptor (CAR) according to claims 1 to 5, an expression vector according to claims 6 and 7, and/or an engineered immune cell according to claims 8 and 9 and a pharmaceutical excipient for use in the treatment of an autoimmune disease.

11. The pharmaceutical composition of claim 10, wherein said autoimmune disease is selected from the group consisting of Cutaneous Lupus Erythemathosus, Dermatomyositis, Psoriasis and systemic sclerosis.
